# EUROPEAN PATENT APPLICATION

(11) **EP 4 019 637 A1**
(43) Date of publication of application: **29.06.2022**
(21) Application number: 20217026.2
(22) Date of filing: 23.12.2020
(51) Int. Cl.: C12N 15/115

(54) **APTAMERS AND USE OF THE APTAMERS IN THE DIAGNOSIS AND TREATMENT OF A SARS-COV-2 INFECTION**

(71) Applicant: Rheinische Friedrich-Wilhelms-Universität Bonn, 53113 Bonn (DE)
(72) Inventor: Mayer, Günter, 53123 Bonn (DE); Famulok, Michael, 53175 Bonn (DE); Schmitz, Anton, 53123 Bonn (DE); Weber, Anna Maria, 53859 Niederkassel (DE); Breuers, Stefan, 53111 Bonn (DE); Fieberg, Volkmar, 57539 Etzbach (DE)
(74) Representative: Michalski Hüttermann & Partner Patentanwälte mbB

(57) **Abstract**

The present invention relates to an aptamer not binding to the receptor-binding domain of the SARS-CoV-2 spike glycoprotein nor inhibiting the interaction of the receptor-binding domain (RBD) of spike glycoprotein with angiotensin-converting enzyme II (ACE2) and/or comprising or consisting of a nucleotide sequence SEQ ID NO: 1, a composition comprising the aptamer, and the use of the aptamer in the detection of SARS-CoV-2 or diagnosis or treatment of a SARS-CoV-2 infection.

## Description

Coronaviruses (CoVs) are respiratory viruses that can cause infections. The recently known coronavirus, SARS-CoV-2, has turned into a global health challenge. The lack of specific treatment and vast and accurate diagnostic systems has made the situation more complicated. Efforts have led to production of several diagnostic kits that are associated with limitations such as inadequate sensitivity and accuracy. Aptamers as multipotent biological probes could be promising candidates to design sensitive and specific biosensors.

It has been identified that SARS CoV-2 infects the human respiratory epithelial cells via interaction of its receptor-binding domain (RBD) of spike glycoprotein (S) with angiotensin-converting enzyme II (ACE2) on the host cells. Like the SARS coronavirus, SARS-CoV-2 uses its spike protein (CoV2-S) to bind to the extracellular domain of the human angiotensin-converting enzyme 2 (ACE2) for initiating the entry into target cells. CoV2-S is a trimeric, highly glycosylated class I fusion protein. It binds to ACE2 via the receptor binding domain (RBD) of its S1 subunit. Consequently, almost all published neutralizing antibodies developed for therapeutic use target RBD. However, mutations in RBD of the SARS-CoV-2 spike glycoprotein can cause RBD-targeted antibodies ineffectual.

Song et al. have isolated novel anti-RBD-SARS-CoV-2 aptamers using ACE2 competition-based aptamer selection strategy and a machine learning screening algorithm, as described in "Discovery of Aptamers Targeting the Receptor-Binding Domain of the SARS-CoV-2 Spike Glycoprotein", Anal. Chem., 2020, 92, 14, 9895-9900. Aptamers are single chained nucleic acids, folding into well-defined three-dimensional shapes based on which they recognise target structures with high affinity and specificity. Aptamers of either single-stranded DNA or RNA can specifically bind to target ligands, such as proteins. Novel diagnostic tools based on aptamers provide high detection sensitivity and specificity to the target agents of SARS-CoV-2, such as the nucleocapsid protein, as described by Chen et al. "A DNA Aptamer Based Method for Detection of SARS-CoV-2 Nucleocapsid Protein", Virologica Sinica, 2020, 35, 351-354. However, the common diagnostic test for COVID-19 is real-time reverse transcription polymerase chain reaction (RT-PCR), and most of these tests are laborious and exhibit long duration. Thus, there is a need for fast and accurate diagnostic tools for the detection of SARS-CoV-2 infections.

Therefore, the object underlying the present invention was to provide improved means for diagnostic or treatment of SARS-CoV-2, particularly aptamers that are usable in diagnostic tools for the detection of SARS-CoV-2.

The problem is solved by an aptamer binding to SARS-CoV-2 spike glycoprotein, wherein the aptamer does not bind to the receptor-binding domain of the SARS-CoV-2 spike glycoprotein nor inhibit the interaction of the receptor-binding domain (RBD) of spike glycoprotein with angiotensin-converting enzyme II (ACE2) and/or comprises or consists of a nucleotide sequence 5'-N₁N₂AN₃GGTAGGTAN4TGCTTGGTAGGGAN₅AN₆TN₇N₈GN₉N₁₀-3' (SEQ ID NO: 1) or a pharmaceutically acceptable salt thereof, wherein:
- N₁, N₂: independently represent G or C,
- N₃, N₄: independently represent T or G,
- N₅: represents T or A,
- N₆, N₇, N₈: independently represent G or C, and
- N₉, N₁₀: independently represent C or T.

Surprisingly, aptamers could be selected that recognise the SARS-CoV-2 spike glycoprotein with high affinity and specificity, but were shown to neither bind to the receptor-binding domain of the SARS-CoV-2 spike glycoprotein nor to inhibit the interaction of the receptor-binding domain (RBD) of spike glycoprotein with angiotensin-converting enzyme II (ACE2). Still, the aptamers were shown to be able to inhibit infection by SARS-CoV-2-S pseudotyped virus and to be usable for detecting a SARS-CoV-2 infection in biological samples. The aptamers were shown to provide high affinity and specificity for the SARS-CoV-2 spike glycoprotein and thus allow a use as biomarkers for diagnosing or detecting SARS-CoV-2 infections. The tests can provide fast and reliable detection of a SARS-CoV-2 infection. As aptamers can be synthesised chemically, aptamer-based tests represent a cost-effective tool that is usable for mass-testing. The aptamers further are usable in the treatment or inhibition of a SARS-CoV-2 infection, such as in nasal sprays. Aptamers can be synthesised chemically and thus meet the requirements for GMP ("Good Manufacturing Practice")-conform manufacture for clinical use more efficiently than for example antibodies that have to be produced in cell cultures.

As used herein, the term "aptamer" refers to a single-stranded oligo(deoxy)nucleotide that recognises its target with high specificity and binds to the target with high affinity in the low nanomolar range. The aptamer can be provided in the form of a single-stranded DNA or preferably chemically stabilized RNA molecule. As will be obvious to a person of ordinary skills in the art, if the nucleic acid is an RNA molecule the thymidine or "T" in the nucleotide sequence is to be read as meaning "U" or uridine. Chemically stabilized RNA molecules may comprise 2'-deoxy-2'-fluoro-pyrimidine, 2'-deoxy-2'-amino-pyrimidine, or 2'-methoxy pyrimidine. Preferably, the aptamer comprises a deoxyribonucleotide sequence. DNA aptamers can exhibit better stability. The aptamer may comprise one or more chemical modifications such as a biotin- or other anchor groups, or fluorophor(s).

Aptamers are provided that comprise or consist of a nucleotide sequence 5'-N₁N₂AN₃GGTAGGTAN4TGCTTGGTAGGGAN₅AN₆TN₇N₈GN₉N₁₀-3' (SEQ ID NO: 1) or a pharmaceutically acceptable salt thereof, wherein:
- N₁, N₂: independently represent G or C,
- N₃, N₄: independently represent T or G,
- N₅: represents T or A,
- N₆, N₇, N₈: independently represent G or C,
- N₉, N₁₀: independently represent C or T.

This motif was found to be important for binding to SARS-CoV-2 spike glycoprotein. Without being bound to a specific theory, it is assumed that the aptamer's binding properties to SARS-CoV-2 spike glycoprotein, which is not a binding to the receptor-binding domain of the SARS-CoV-2 spike glycoprotein and does not inhibit the interaction of the RBD with ACE2 depends on the presence of this minimal binding motif.

In embodiments, an aptamer binding to SARS-CoV-2 spike glycoprotein is provided where the aptamer does not bind to the receptor-binding domain of the SARS-CoV-2 spike glycoprotein nor inhibit the interaction of the receptor-binding domain (RBD) of spike glycoprotein with angiotensin-converting enzyme II (ACE2). In embodiments, an aptamer is provided comprising or consisting of a nucleotide sequence of SEQ ID NO: 1. In embodiments is provided an aptamer binding to SARS-CoV-2 spike glycoprotein where the aptamer comprises or consists of a nucleotide sequence of SEQ ID NO: 1 and does not bind to the receptor-binding domain of the SARS-CoV-2 spike glycoprotein nor inhibit the interaction of the receptor-binding domain (RBD) of spike glycoprotein with angiotensin-converting enzyme II (ACE2).

In embodiments, N₁ and N₂ may represent C, N₃ and N₄ may represent G, N₅ may represent T, N₆, N₇ and N₈ may represent G, N₉ may represent C or T, and N₁₀ may represent T. Particularly embodiments with N₉ representing C include aptamers which exhibit specific binding to SARS-CoV-2 spike glycoprotein. In such embodiments, the aptamer comprises or consists of a nucleotide sequence 5'-CCATGGTAGGTATTGCTTGGTAGGGATAGTGGGCT-3' (SEQ ID NO: 2) or a pharmaceutically acceptable salt thereof.

In other embodiments, N₁, N₂, N₃ and N₄ may represent G, N₅ may represent A, N₆, N₇ and N₈ may represent C, N₉ may represent T or C, and N₁₀ may represent C. Particularly embodiments with N₉ representing C include aptamers which exhibit specific binding to SARS-CoV-2 spike glycoprotein. In such embodiments, the aptamer comprises or consists of a nucleotide sequence 5'-GGAGGGTAGGTAGTGCTTGGTAGGGAAACTCCGCC-3' (SEQ ID NO: 3) or a pharmaceutically acceptable salt thereof.

Without being bound to a specific theory, it is assumed that the motif of SEQ ID NO: 1, and 2 and 3 is important for forming the three-dimensional stem-loop structure of the aptamer, particularly of a stem structure. The 5'- and/or the 3'-ends of the aptamer may comprise further nucleotides. A longer sequence may improve binding or affinity of the aptamer. Without being bound to a specific theory, it is further assumed that improved binding and affinity may be due to an improved three-dimensional stem-loop structure of the aptamer, such as a better forming of a putative apical stem structure. It is further assumed that the longer a sequence is, the more a deletion or a point mutation in the sequence may be tolerated without loss of the ability to bind to SARS-CoV-2 spike glycoprotein.

In embodiments, the aptamer comprises or consists of a nucleotide sequence or a pharmaceutically acceptable salt thereof selected from the group comprising:
5'-CCCATGGTAGGTATTGCTTGGTAGGGATAGTGGGCTTGATG-3' (SEQ ID NO: 4),
5'-CCATGGTAGGTATTGCTTGGTAGGGATAGTGGGCTTGATG-3' (SEQ ID NO: 5), and
5'-CCCATGGTAGGTATTGCTTGGTAGGGATAGTGGGTTTGATG-3' (SEQ ID NO: 6). It was shown that aptamers comprising such a sequence allow for highly specific binding to SARS-CoV-2 spike glycoprotein in concentrations as low as 10-20 nM.

In further embodiments, the aptamer comprises or consists of a nucleotide sequence or a pharmaceutically acceptable salt thereof selected from the group comprising:
5'-AGGAGGGTAGGTAGTGCTTGGTAGGGAAACTCCGCCGATT-3' (SEQ ID NO: 7),
5'-AAGGAGGGTAGGTAGTGCTTGGTAGGGAAACTCCGCCGATT-3' (SEQ ID NO: 8),
5'-AAGGAGGGTAGGTAGTGCTTGGTAGGGAAACTCCGCCGAT-3' (SEQ ID NO: 9),
5'-AGGAGGGTAGGTAGTGCTTGGTAGGGAAACTCCGTCGATT-3' (SEQ ID NO: 10), and
5'-AAGGAGGGTAGGTAGTGCTTGGTAGGGAAACTCCGTCGAT-3' (SEQ ID NO: 11).

It was shown that aptamers comprising such a sequence allow for highly specific binding to SARS-CoV-2 spike glycoprotein with good K_{D} values.

In a preferred embodiment, the aptamer comprises or consists of a nucleotide sequence selected from the group of: 5'-GGGAGAGGAGGGAGATAGATATCAACCCATGGTAGGTATTGCTTGGTAGGGATAGTGGG CTTGATGTTTCGTGGATGCCACAGGAC-3' (SEQ ID NO: 12), 5'-GATATCAACCCATGGTAGGTATTGCTTGGTAGGGATAGTGGGCTTGATGTT-3' (SEQ ID NO: 13),
5'-ATCAACCCATGGTAGGTATTGCTTGGTAGGGATAGTGGGCTTGAT-3' (SEQ ID NO: 14), 5'-CAACCCATGGTAGGTATTGCTTGGTAGGGATAGTGGGCTTG-3' (SEQ ID NO: 15) and 5'-CCCATGGTAGGTATTGCTTGGTAGGGATAGTGGG-3' (SEQ ID NO: 16) or a pharmaceutically acceptable salt thereof. It was shown that these aptamers exhibited favorable specificity in binding to SARS-CoV-2 spike glycoprotein in *in vitro* testing and that the aptamer of SEQ ID NO: 12 also provided effective inhibition of infection by SARS-CoV-2-S pseudotyped virus.

In a further preferred embodiment, the aptamer comprises or consists of a nucleotide sequence 5'-GGAGGGTAGGTAGTGCTTGGTAGGGAAACTCC-3' (SEQ ID NO: 17) or a pharmaceutically acceptable salt thereof. It was shown that also this aptamers exhibited favorable specificity in binding to SARS-CoV-2 spike glycoprotein in *in vitro* testing.

In further preferred embodiments, the aptamer comprises or consists of a nucleotide sequence 5'-GGGAGAGGAGGGAGATAGATATCAACCATGGTAGGTATTGCTTGGTAGGGATAGTGGGC TTGATGTTTCGTGGATGCCACAGGAC-3' (SEQ ID NO: 18), 5'-GGGAGAGGAGGGAGATAGATATCAAAGGAGGGTAGGTAGTGCTTGGTAGGGAAACTCCG CCGATTTTTCGTGGATGCCACAGGAC-3' (SEQ ID NO: 19), 5'-GGGAGAGGAGGGAGATAGATATCAAAAGGAGGGTAGGTAGTGCTTGGTAGGGAAACTCC GCCGATTTTTCGTGGATGCCACAGGAC-3' (SEQ ID NO: 20), or a pharmaceutically acceptable salt thereof. It was shown that also these aptamers exhibited good specificity in binding to SARS-CoV-2 spike glycoprotein in *in vitro* testing.

In embodiments, the aptamer is part of a dimer, trimer or multimer comprising two, three or multiple nucleotide sequences selected from the group comprising SEQ ID NOs: 1-20, wherein the nucleotide sequences are connected by a linker. Preferably, the aptamer is a trimer. The SARS-CoV-2 spike glycoprotein is a trimeric protein. A trimeric aptamer thus can provide binding to all three binding sites on the spike glycoprotein.

The two, three or multiple nucleotide sequences of SEQ ID NOs: 1-20 may be identical, or the dimer, trimer or multimer may comprise aptamers of different sequences. The aptamer sequences may be selected from the group of SEQ ID NO: 1, SEQ ID NO: 2, SEQ ID NO: 3, SEQ ID NOs: 4-6, SEQ ID NOs: 7-11, or SEQ ID NO: 12-16.

The linker may be selected from a nucleotide linker, polyethylene glycol (PEG), a peptide linker, an alkyl linker or an aminoalkyl linker. The linker may be a nucleotide linker. Nucleotide linkers can easily be included during the synthesis of a dimer, trimer or multimer. The nucleotides of the linker may be selected from the group comprising guanosine, cytidine, adenosine and/or thymidine. The linker may have a random sequence. A preferred nucleotide is adenosine. Especially preferred linkers are poly A-linkers. Such linkers are easy to synthesize and avoid random forming of secondary structures. In other embodiments, the linker may be a polyethylene glycol (PEG) linker. The aptamer may be conjugated to the polyethylene glycol (PEG) molecules via covalent linkage, non-covalent linkage, or both. Methods for manufacturing pegylated oligonucleotides and PEGylation reagents of defined numbers of PEG units are known in the art. The PEG moiety conjugated to the aptamer can be linear or branched, preferably linear. Polyethylene glycol is a biologically inert, non-immunogenic compound, and is non-toxic and highly flexible. In further embodiments the linker may be a linear or branched, saturated or unsaturated alkyl group. In other embodiments, the linker may be an aminoalkyl linker. Such linkers can be easily synthesized. In other embodiments the linker may be a peptide linker, wherein the peptide linker preferably comprises amino acids selected from the group of glycine, alanine and/or β-alanine. Preferably, a peptide linker comprises β-β-alanine units.

The aptamers also are usable in form of pharmaceutically acceptable salts thereof. The term "pharmaceutically acceptable salts" refers to salts prepared from pharmaceutically acceptable non-toxic bases or acids. A pharmaceutically acceptable salt can be conveniently prepared from pharmaceutically acceptable non-toxic bases, including inorganic bases and organic bases. Preferred salts derived from inorganic bases include ammonium, calcium, magnesium, potassium and sodium salts. Salts derived from pharmaceutically acceptable organic non-toxic bases include salts of primary, secondary, and tertiary amines, as well as cyclic amines. Preferably, the pharmaceutically acceptable salt is selected from the group of sodium or potassium salts. Also, calcium or magnesium salts can be preferred.

A further aspect of the present invention relates to an aptamer according to the invention, for use as a medicament or a diagnostic reagent. For the description of the aptamers, reference is made to the description above. The aptamer binds to SARS-CoV-2 spike glycoprotein, wherein the aptamer does not bind to the receptor-binding domain of the SARS-CoV-2 spike glycoprotein nor inhibit the interaction of the receptor-binding domain (RBD) of spike glycoprotein with angiotensin-converting enzyme II (ACE2) and/or comprises or consists of a nucleotide sequence of SEQ ID NO: 1. In preferred embodiments, the aptamer comprises or consists of a nucleotide sequence selected from the group of SEQ ID NO: 2, SEQ ID NO: 3, SEQ ID NOs: 4-6, SEQ ID NOs: 7-11, or SEQ ID NO: 12-20, or pharmaceutically acceptable salts thereof, preferably of a nucleotide sequence SEQ ID NO: 1, SEQ ID NO: 2, SEQ ID NO: 4, or SEQ ID NO: 12-16.

The aptamers advantageously are able to recognise SARS-CoV-2 spike protein with high affinity and specificity and low K_{D} values in biological samples. This allows that the aptamers can be used for the detection of SARS-CoV-2 and suggests a use in the diagnosis of a SARS-CoV-2 infection. Hence, the aptamer is useful as a diagnostic reagent.

In preferred embodiments, the aptamer is for use in the detection of SARS-CoV-2 or the diagnosis of a SARS-CoV-2 infection. The aptamer allows detecting binding to SARS-CoV-2 spike protein with high affinity and specificity in biological samples and thus a use in highly sensitive diagnostic tools to be used for an early and fast testing. The aptamers, for example, may be used as a diagnostic reagent in lateral flow assays (LFA) or in enzyme-linked oligonucleotide assays (ELONA). The aptamers may help to improve fast and cost-sensitive testing and mass-testing of large cohorts. This would provide vast benefits in identifying infected patients and improve fast isolation of infections.

The aptamer advantageously was able to inhibit infection by SARS-CoV-2-S pseudotyped virus. In other preferred embodiments, the aptamer thus is for use in the treatment of a SARS-CoV-2 infection. The aptamer was found to reproducibly inhibit infection by SARS-CoV-2-S pseudotyped virus. This provides a huge advantage, as targeting SARS-CoV-2 infections in a fast and easy to handle manner is a major challenge in treatment and inhibition of SARS-CoV-2 infections.

For use as a medicament or a diagnostic reagent the aptamers can be used or included in a composition. For use as a medicament the aptamer can be used or included in a pharmaceutical composition. For use as a diagnostic reagent the aptamer can be used or included in a diagnostic composition. Accordingly, a further aspect relates to a diagnostic or pharmaceutical composition comprising an aptamer according to the invention. For the description of the aptamers, reference is made to the description above. The aptamer binds to SARS-CoV-2 spike glycoprotein, wherein the aptamer does not bind to the receptor-binding domain of the SARS-CoV-2 spike glycoprotein nor inhibit the interaction of the receptor-binding domain (RBD) of spike glycoprotein with angiotensin-converting enzyme II (ACE2) and/or comprises or consists of a nucleotide sequence of SEQ ID NO: 1. In preferred embodiments, the aptamer comprises or consists of a nucleotide sequence selected from the group of SEQ ID NO: 2, SEQ ID NO: 3, SEQ ID NOs: 4-6, SEQ ID NOs: 7-11, or SEQ ID NO: 12-20, or pharmaceutically acceptable salts thereof, preferably of a nucleotide sequence SEQ ID NO: 2, SEQ ID NO: 4, or SEQ ID NO: 12-16. In preferred embodiments, the diagnostic or pharmaceutical composition is for use in the detection of SARS-CoV-2 or diagnosis or treatment of a SARS-CoV-2 infection.

The diagnostic composition, in embodiments, may be used in a lateral flow assay (LFA) or in an enzyme-linked oligonucleotide assay (ELONA). Such test are usable for rapid testing and are able to provide faster and more cost-sensitive testing than current PCR-based or antibody-based test systems. When the aptamer is brought into contact with a sample, it will bind specifically to SARS-CoV-2 spike glycoprotein present in the sample. For diagnostic purposes, the aptamer may comprise a labelling which provides that the bound aptamer can be detected by determining the presence or absence of a signal provided by the label. For example, the aptamer can be labelled with a fluorescent dye. A fluorescence-labelling, for example provided by a fluorescence dye, can provide a visualisation of the bound aptamer by fluorescence or laser scanning microscopy or flow cytometry. Further, particularly for detection purposes, the aptamer can be immobilised on conventional supports such as beads providing a tool for the detection of aptamer-bound SARS-CoV-2 and thus diagnosing infection. Further, the aptamer can be biotinylated or coupled to streptavidin, avidin or neutravidin for use in the specific detection of SARS-CoV-2 spike glycoprotein.

Apart from being useful for detecting or diagnosing a SARS-CoV-2 infection, the aptamers also are applicable for a treatment and particularly for inhibition of infections with SARS-CoV-2. For use as a medicament the aptamers can be used or included in a pharmaceutical composition. The compositions can be suitable for parenteral administration, including subcutaneous, intramuscular, intravenous administration and inhalation. Compositions suitable for parenteral administration may be prepared as solutions or suspensions of the aptamer in water or isotonic buffers. Compositions suitable for injectable use include sterile aqueous solutions or dispersions. The compositions can be suitable for inhalation therapy and administration via inhalation. In embodiments, the pharmaceutical composition may be in the form of a nasal spray. Compositions suitable for administration via inhalation may be prepared as solutions or suspensions of the aptamer in water or isotonic buffers. In embodiments, the pharmaceutical composition may be in the form a nasal spray. Nasal sprays are easy to apply and a preferred method of targeting infections with SARS-CoV-2.

By binding to SARS-CoV-2 spike glycoprotein the aptamer is able to inhibit or prevent infections. Accordingly, the pharmaceutical composition can comprise an aptamer according to the invention as an active ingredient.

The aptamers however also are usable as a vehicle for delivery of other therapeutics, such as anti-SARS-CoV-2 drugs, toxins, antiviral compounds or antibodies, to the virus-infected cells. SARS-CoV-2 antiviral drugs, toxins or antibodies can be attached to the aptamers to be delivered to virus-infected cells. The pharmaceutical composition comprising the aptamers thus also may be useful as a molecular vehicle for delivery of cargo such as anti-viral drugs or antibodies agents to the virus-infected cells. Compounds, drugs or antibodies can be directly coupled to the aptamer, in a covalent or non-covalent fashion. Alternatively, the aptamer can be attached to the surface of a liposome containing anti-viral drugs or to nanoparticles encapsulating an anti-viral agent. The aptamers may provide a SARS-CoV-2-specific drug delivery composition comprising an aptamer and an anti-viral agent such as an antibody.

The composition may be produced under sterile conditions using standard pharmaceutical techniques well known to those skilled in the art. For compositions convenient pharmaceutical media may be employed. For example, water, glycols, oils, alcohols and the like may be used to form liquid preparations such as solutions. The composition may comprise a pharmaceutical carrier, which can be, for example, a solid, liquid, or gas. Suitable carriers preferably are liquid and correspond to the substances ordinarily employed in formulation technology for pharmaceutical formulations.

The present invention also relates to the use of an aptamer according to the invention, for the manufacture of a medicament or a diagnostic reagent, particularly for use in the detection of SARS-CoV-2 or diagnosis or treatment of a SARS-CoV-2 infection. For the description of the aptamers, reference is made to the description above. The aptamer binds to SARS-CoV-2 spike glycoprotein, wherein the aptamer does not bind to the receptor-binding domain of the SARS-CoV-2 spike glycoprotein nor inhibit the interaction of the receptor-binding domain (RBD) of spike glycoprotein with angiotensin-converting enzyme II (ACE2) and/or comprises or consists of a nucleotide sequence of SEQ ID NO: 1. In preferred embodiments, the aptamer comprises or consists of a nucleotide sequence selected from the group of SEQ ID NO: 2, SEQ ID NO: 3, SEQ ID NOs: 4-6, SEQ ID NOs: 7-11, or SEQ ID NO: 12-20, or pharmaceutically acceptable salts thereof, preferably of a nucleotide sequence SEQ ID NO: 2, SEQ ID NO: 4, or SEQ ID NOs: 12-16.

A further aspect relates to an *in vitro* method of detecting SARS-CoV-2 or diagnosing a SARS-CoV-2 infection, the method comprising the step of detecting the binding of an aptamer to SARS-CoV-2 spike glycoprotein in a sample obtained from a subject. For the description of the aptamers, reference is made to the description above. The aptamer binds to SARS-CoV-2 spike glycoprotein, wherein the aptamer does not bind to the receptor-binding domain of the SARS-CoV-2 spike glycoprotein nor inhibit the interaction of the receptor-binding domain (RBD) of spike glycoprotein with angiotensin-converting enzyme II (ACE2) and/or comprises a nucleotide sequence of SEQ ID NO: 1. In preferred embodiments, the aptamer comprises or consists of a nucleotide sequence selected from the group of SEQ ID NO: 2, SEQ ID NO: 3, SEQ ID NOs: 4-6, SEQ ID NOs: 7-11, or SEQ ID NO: 12-20, or pharmaceutically acceptable salts thereof, preferably of a nucleotide sequence SEQ ID NO: 2, SEQ ID NO: 4, or SEQ ID NOs: 12-16.

As used herein, the term "sample" refers to any material, which probably contains coronaviruses, particularly SARS-CoV-2, including any liquid or fluid sample or solid material, particularly a sample derived from a biological source such as a patient or test subject. The term sample particularly refers to biological material, for example cells or tissues, biological fluids or supernatants. The sample for example can comprise cells from a body fluid of a possibly infected subject, preferably a human. The sample can be a body fluid such as blood, serum, plasma, saliva, phlegm and urine. The sample preferably is selected from saliva of a patient, preferably a human. The method comprises bringing the aptamer into contact with a sample, which probably contains coronaviruses, particularly SARS-CoV-2. Determination of virus in saliva samples is commonly used for SARS-CoV-2 testing.

A further aspect of the present invention relates to a method of treating a SARS-CoV-2 infection, the method comprising the step of administering to a subject a therapeutically effective amount of an aptamer or a pharmaceutical composition according to the invention. The term "therapeutically effective amount" is used herein to mean an amount or dose sufficient to cause an improvement in a clinically significant condition in the subject.

Unless otherwise defined, the technical and scientific terms used herein have the same meaning as commonly understood by one of ordinary skill in the art to which this invention belongs.

The Examples, which follow serve to illustrate the invention in more detail but do not constitute a limitation thereof.

The figures show:
- Figure 1: results of the screening of the DNA of selection cycles 1 (R1) and 12 (R12) targeting SARS-CoV-2 spike, Erk2, and Dectin-1.
- Figure 2: results of the screening of the DNA of the starting library (SL), selection cycle 12 (R12), sequences SP1-8 targeting SARS-CoV-2 spike, and SP5sc.
- Figure 3: results of the screening of the sequences SP5-8 and SP5sc targeting SARS-CoV-2 spike, SARS-CoV-1 spike, RBD and ACE2.
- Figure 4: results of the screening of the aptamers SP5-7 targeting SARS-CoV-2 spike compared to truncated aptamers and sequences with one point mutation.
- Figure 5: results of the ELONA assay using the 5'-biotinylated aptamers SP6 and SP6.34, and non-binding control sequences and SARS-CoV-2 spike.
- Figure 6: results of the affinity determination for 5'-biotinylated full-length aptamers SP5, SP6 and SP7 at 37°C in Figures 6 A), C), E) and at 25°C in Figures 6 B), D) and F).
- Figure 7: Coomassie-stained protein gels illustrating that the interaction between SARS-CoV-2 spike and ACE2 was not affected by aptamer SP6.
- Figure 8: infection results of aptamer SP6 for CoV2-S pseudotype and VSV-G pseudotype.

### Materials and Methods

### Coupling of SARS-CoV-proteins to Dynabeads His-Tag isolation & pulldown:

For immobilization of SARS-CoV-proteins, Dynabeads His-Tag Isolation & Pulldown (ThermoFisher) were used. For this purpose, 9.6 nmol of SARS-CoV-proteins, prepared in 1 mL wash/binding buffer (50 mM Sodium-Phosphate, pH 8.0, 300 mM NaCl, 0.01% Tween^{®}-20) were coupled to 100 µL of bead solution (40 mg beads/mL), according to the manufacturer's protocol. The provided buffer, by the manufacturer, of the bead solution was discarded by separation before coupling, using a DynaMag^{™}-2 Magnet (ThermoFisher). For the coupling reaction, the solution was incubated for 30 min on 4°C, using a Tube Revolver Model D-6050 (neoLab) rotating at a speed of 20 rpm. According to the manufacturer's protocol, three washing steps with 1 mL wash/binding buffer were carried out, followed by one additional washing step with storing buffer (ssDNA selection = 1.25× PBS; 171.25 mM NaCl (Fisher Scientific), 3.38 mM KCl (Roth), 12.5 mM Na₂HPO₄ (Roth), 2.2 mM KH₂PO₄ (Roth), pH 7.4; 1 mg/mL Albumin (BSA) Fraction V (pH 7.0) (AppliChem); 3.25 mM MgCl₂ // 2'fRNA selection = 1.25× PBS; 171.25 mM NaCl (Fisher Scientific), 3.38 mM KCl (Roth), 12.5 mM Na₂HPO₄ (Roth), 2.2 mM KH₂PO₄ (Roth), pH 7.4; 1 mg/mL Albumin (BSA) Fraction V (pH 7.0) (AppliChem)), before resuspending SARS-CoV2-protein beads in 1 mL of storing buffer. In the particular case of competitor, 0.125 mg/mL Heparin was added to the storing buffer.

### Automated selection:

### ssDNA selection

For the ssDNA selection, a D2 DNA library (5'-GGGAGAGGAGGGAGATAGATATCAA-N40-TTTCGTGGATGCCACAGGAC-3', (SEQ ID NO: 21)) and D2 primers were used, synthesized by Ella Biotech GmbH (Munich, Germany). For amplifying the library by PCR, the following primers were used: forward primer, including a Cyanin dye (Cy5) modification at the 5' end (Cy5-D2 fw) 5' Cy5 - GGGAGAGGAGGGAGATAGATATCAA-3' (SEQ ID NO: 22) and reverse primer, including a phosphate modification at the 5' end (Phos-D2 rv) 5' P-GTCCTGTGGCATCCACGAAA-3' (SEQ ID NO: 23) in PCR master mix (1× colorless GoTaq^{®} Flexi Buffer (Promega), 2 mM MgCl₂ (Roth), 0.2 mM dNTP (Genaxxon) each). The PCR reaction was performed by using GoTaq^{®} G2 Flexi DNA Polymerase (Promega) for amplification, including 1 µM of Cy5-D2 fw and Phos-D2 rv primers each, in a total reaction volume of 100 µL. Cycling the PCR program (30 s 95 °C, 30 s 62 °C, 30 s 72 °C; hold 10 °C) in a TRobot thermal cycler (Biometra) in the first four selection rounds for 18 PCR cycles and in all following selection rounds after for 16 PCR cycles. For all steps performed on the TRobot, an arched auto-sealing lid (Bio-Rad) was used to seal the reaction plate. 1 µL GoTaq^{®} G2 Flexi DNA Polymerase (Promega) was added to start the PCR reaction. For generating ssDNA after PCR, a lambda exonuclease digestion was performed, using 2µL lambda exonuclease (ThermoFisher).

The automated pipetting steps were performed by a Biomek NX^{P} (Beckman Coulter). Initializing the automated selection with an incubation of 0.5 nmol of D2 ssDNA library pipetted to the SARS-CoV-proteins immobilized on Dynabeads His-Tag Isolation & Pulldown (ThermoFisher) for 30 min at 37 °C while shaking at a speed of 700 rpm; pipetting up and down every 5 min during incubation. For a final concentration in the selection of 3 mM MgCl₂ and 1× PBS, the initial 0.5nmol D2 ssDNA library was prepared with MgCl₂ (Roth) and phosphate-buffered saline (PBS; 137 mM NaCl (Fisher Scientific), 2.7 mM KCl (Roth), 10 mM Na₂HPO₄ (Roth), 1.76 mM KH₂PO₄ (Roth), pH 7.4). Followed by a washing step after incubation, the samples were washed two times with wash buffer (3 mM MgCl₂, 1× PBS), increasing the number of washing steps every selection round by two more washes, until reaching a total of eight washes per selection round. Prior to PCR, the ssDNA was recovered by incubation of 5 min at 80 °C in a TRobot thermal cycler using double distilled H₂O (TKA Wasseraufbereitungssysteme GmbH). After PCR, a lambda exonuclease digestion followed to generate ssDNA by incubating 2µL lambda exonuclease with 30µL of PCR product for 60 min at 37 °C. 20µL of the ssDNA were pipetted into 80µL of beads suspension to start the next selection round.

### Agarose gel analysis:

Agarose LE (Genaxxon) was used to perform a 4% agarose Gel and was pre-stained with ethidium bromide (Roth). 1 µL 6x DNA Loading Dye (Thermo Scientific) with 5 µL of dsDNA were mixed and loaded on the gel. As reference 4 µL of GeneRuler Ultra Low Range DNA Ladder (Thermo Scientific) were loaded on the gel. A Genoplex system (VWR) was used for visualization of the DNA.

### Next-generation sequencing (NGS):

The starting library and enriched libraries of cycle 3 to 12 were analyzed by NGS using the Illumina HiSeq1500 platform. Samples were prepared according to Tolle and Mayer (2016). "Preparation of SELEX Samples for Next-Generation Sequencing." Methods Mol Biol 1380: 77-84. Briefly, 12 different index primers were attached to the different libraries via PCR, allowing the analysis of 12 samples in parallel on one lane. PCR products were purified using the NucleoSpin Clean-Up kit (Macherey Nagel), and equal amounts of PCR product of each library were mixed to a final amount of 2 µg DNA. For the hybridization to the flow cell a subsequent adapter ligation step was performed according to the manufacturer's instructions (TruSeq DNA PCR-Free Sample Preparation Kit LT, Illumina). Samples were purified by agarose gel purification.

The NGS data was analyzed using the inhouse AptaNext software and MEME suite (Bailey, T. L. et al. (2009). "MEME SUITE: tools for motif discovery and searching." Nucleic Acids Res 37(Web Server issue): W202-20). Five families from cycle 12 were identified and the most abundant sequences were tested in a FACS binding assay. Secondary structures of the aptamers were predicted with Mfold (Zuker, M. (2003). "Mfold web server for nucleic acid folding and hybridization prediction." Nucleic Acids Res 31(13): 3406-341).

### Constructs and plasmids:

Plasmids for SARS-CoV-2-Se, SARS-CoV-2-S-HexaPro and SARS-CoV-Se (kindly provided by Jason McLellan, The University of Texas at Austin, USA) code for the prefusion-stabilized ectodomains of the S proteins and carry on the C-terimus a trimerization motif, a HRV 3C cleavage site, 8xHis and TwinStrep tags [Wrapp et al. (2020) Cryo-EM structure of the 2019-nCoV spike in the prefusion conformation. Science 367, 1260-1263]. SARS-CoV-2-S(D614G)-Δ19 codes for the S protein (GenBank NC_045512) containing the D614G mutation and lacking the C-terminal 19 amino acids thus deleting the ER-retention motif and enhancing transport to the plasma membrane. SARSCoV-2-S-RBD codes for amino acids 319 - 591 of the S protein and contains the signal peptide of the S protein on the N-terminus to allow secretion and a HRV 3C cleavage site, 8xHis and TwinStrep tags on the C-terminus. ACE2e contains, after cleavage of the signal peptide, amino acids 19 - 615 of human ACE2 (UniProt Q9BYF1), an N-terminal myc tag and a C-terminal HRV 3C cleavage site followed by an 8xHis tag. The myc and ACE2 coding sequence was amplified form pCEP4-myc-ACE2 (addgene #141185) [Chan et al. (2020). Engineering human ACE2 to optimize binding to the spike protein of SARS coronavirus 2. Science 369, 1261-1265]. All these proteins were cloned into pCAG which is based on pCAGGS only lacking the SV40 ori of the latter. All constructs were assembled from PCR-amplified fragments using Q5 DNA Polymerase (NEB) or synthetic genes (Eurofins) except for the pCAG backbone which was linearized by restriction digestion. For assembly the NEBuilder HiFi DNA Assembly Master Mix was used (NEB). Coding sequences of all constructs were verified by Sanger sequencing (Eurofins).

### Protein expression and purification:

Proteins were expressed in FreeStyle 293F cells (Thermo). 293F cells at 1x10⁶ cells / ml in FreeStyle 293 Expression Medium (Thermo) were transfected with 1 mg plasmid and 2 mg PEI max (Polysciences) per liter of cells. 3 - 5 days after transfection proteins were purified from the culture medium after removing cells and debris by centrifugation (10 min, 800 g, rt, followed by 30 min, 10000 g, 4 °C). The cleared medium was adjusted to 50 mM HEPES/KOH, pH 7.8 / 300 mM NaCl / 25 mM imidazole and loaded overnight onto a column containing 2 ml Protino Ni-NTA Agarose (Macherey-Nagel) per liter of medium. After washing with 50 mM HEPES/KOH, pH 7.8 / 300 mM NaCl / 25 mM imidazole proteins were eluted in the same buffer containing 1 M imidazole. Eluted proteins were concentrated using Vivaspin Turbo concentrators (Sartorius) and loaded on a Superose 6 column (Cytiva) equilibrated in 20 mM HEPES/KOH, pH 7.8 / 150 mM NaCl to remove aggregated material. Peak fractions were pooled, concentrated, flash-frozen in liquid nitrogen and stored at -80 °C.

### Pseudovirus generation:

VSV pseudotypes were generated as published by Hoffmann et al. (2020) SARS-CoV-2 Cell Entry Depends on ACE2 and TMPRSS2 and Is Blocked by a Clinically Proven Protease Inhibitor. Cell 181, 271-280. Briefly, Hek293T cells transfected with pCAG-SARS-CoV-2-S(D614G)-Δ19 or pcDNA3.1-VSV-G, respectively, were inoculated with VSV-ΔG* (kindly provided by Gert Zimmer, Institute of Virology and Immunology, Mittelhäusern, Switzerland). In VSV-ΔG* the VSV-G open reading frame is replaced by an expression cassette for GFP and firefly luciferase [Berger Rentsch, M., Zimmer, G. (2011) A Vesicular Stomatitis Virus Replicon-Based Bioassay for the Rapid and Sensitive Determination of Multi-Species Type I Interferon. PLoS One 6, e25858. doi:10.1371/journal.pone.0025858] allowing infected cells to be detected by GFP fluorescence or luciferase activity. After 1 h incubation at 37 °C the inoculum was removed, the cells were washed with DMEM and cultivated for 16 - 18 h in DMEM / 2 % FBS / 30 mM Hepes at 34 °C. The culture medium containing the pseudotyped particles was clarified from cellular debris by centrifugation (800 g, 5 min, rt). Aliquots were flash-frozen in liquid nitrogen and stored at -80 °C.

### Example 1

### Selection of aptamers

Aptamers were selected using automated selection as described above. For this, the trimerized His-tagged extracellular domain of CoV2-S, stabilized in the prefusion conformation, was expressed and purified from HEK293 cells and immobilized for the selection on magnetic beads. After 12 rounds of selection, the selected ssDNA pool was PCR-amplified.

### Example 2

### Determination of DNA binding targeting SARS-CoV-2 spike, Erk2 and Dectin-1

The binding of the DNA of selection cycles 1 (R1) and 12 (R12) targeting SARS-CoV-2 spike, Erk2 and Dectin-1 immobilized on magnetic beads was determined by FACS analysis. The proteins were immobilized on magnetic particles (ThermoFisher) via His-Tag as done for the selection and stored in 1.25× PBS + 1 mg/ml BSA at 4°C. 5'-Cy5-labeled ssDNA libraries were prepared and incubated at a concentration of 500 nM with 4 µl bead suspension in a final volume of 10 µl 1× PBS with 3 mM MgCl2 and 0.8 mg/ml BSA at 37°C and 650 rpm. After removal of the supernatant, beads were washed two times with 100 µl 1× PBS with 3 mM MgCl2 and resuspended in 100 µl 1× PBS with 3 mM MgCl2 for analysis by flow cytometry. Approximately 20,000 events were analyzed for each sample.

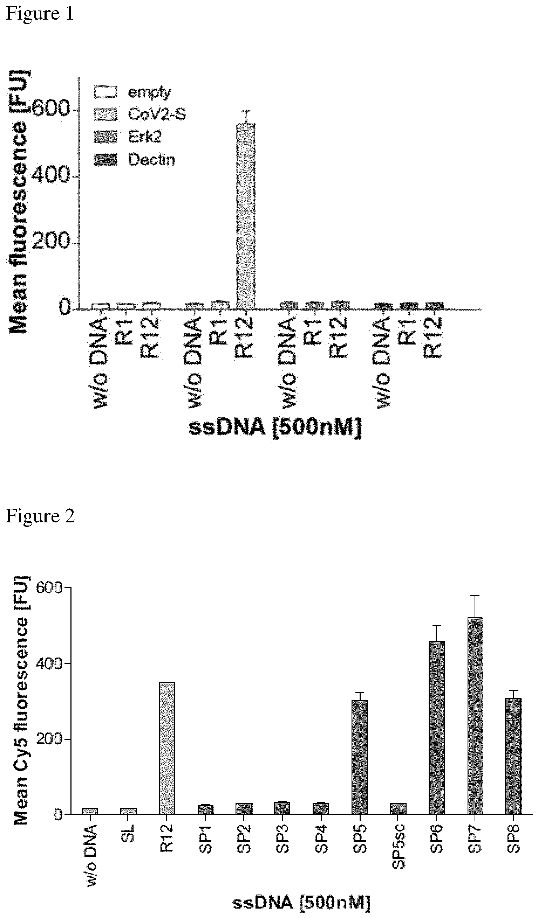

Figure 1 illustrates the results of the screening of the DNA of selection cycles 1 (R1) and 12 (R12) targeting SARS-CoV-2 spike (CoV2-S), Erk2 and Dectin-1. As can be taken from Figure 1, DNA of cycle 12 (R12) selectively binds SARS-CoV-2 spike, whereas no binding was determined for DNA of cycle 1 for any of the proteins and DNA of cycle 12 for Erk2 and Dectin.

The enriched DNA populations were subjected to next-generation sequencing (NGS), in which 10⁶ to 10⁷ sequences were analyzed per selection cycle. Sequence analysis provided 8 candidate molecules having a nucleotide sequence as follows:

SP1: 5'-
GGGAGAGGAGGGAGATAGATATCAAGGGCGGAGGAGGGGGCCACACCAAAACACG
TTCAACTTTTCGTGGATGCCACAGGAC-3' (SEQ ID NO: 24)

SP2: 5'-
GGGAGAGGAGGGAGATAGATATCAAGGGAGGGAGGGTGGGGGTTCTCGCTGCGGGTTT
TGGTGCTTTCGTGGATGCCACAGGAC-3' (SEQ ID NO: 25)

SP3: 5'-
GGGAGAGGAGGGAGATAGATATCAAATCGGGGGGTGGGTTTGGGTATGGGGTCTGCACT
ATGGCTTTTCGTGGATGCCACAGGAC-3' (SEQ ID NO: 26)

SP4: 5'-
GGGAGAGGAGGGAGATAGATATCAATCGCGGGGGCGGTCGGTGCTCGTTCGAGGGG
TCGCAGTTTCGTGGATGCCACAGGAC-3' (SEQ ID NO: 27)

SP5: 5'-
GGGAGAGGAGGGAGATAGATATCAACCATGGTAGGTATTGCTTGGTAGGGATAGTGGGC
TTGATGTTTCGTGGATGCCACAGGAC-3' (SEQ ID NO: 18)

SP6: 5'-
GGGAGAGGAGGGAGATAGATATCAACCCATGGTAGGTATTGCTTGGTAGGGATAGTGGG
CTTGATGTTTCGTGGATGCCACAGGAC-3' (SEQ ID NO: 12)

SP7: 5'-
GGGAGAGGAGGGAGATAGATATCAAAGGAGGGTAGGTAGTGCTTGGTAGGGAAACTCCG
CCGATTTTTCGTGGATGCCACAGGAC-3' (SEQ ID NO: 19)

SP8: 5'-
GGGAGAGGAGGGAGATAGATATCAAAAGGAGGGTAGGTAGTGCTTGGTAGGGAAACTCC
GCCGATTTTTCGTGGATGCCACAGGAC-3' (SEQ ID NO: 20).

### Example 3

### Determination of aptamer binding targeting SARS-CoV-2 spike

The binding of the DNA of the starting library (SL) selection cycle 12 (R12), aptamer sequences SP1-8 and a non-binding scrambled control sequences SP5sc targeting SARS-CoV-2 spike immobilized on magnetic beads was investigated by FACS analysis according to example 2 to identify SARS-CoV-2 spike binding aptamers.

The following aptamers were determined:
SP1: (SEQ ID NO: 24)
SP2: (SEQ ID NO: 25)
SP3: (SEQ ID NO: 26)
SP4: (SEQ ID NO: 27)
SP5: (SEQ ID NO: 18)
SP6: (SEQ ID NO: 12)
SP7: (SEQ ID NO: 19)
SP8: (SEQ ID NO: 20)

Figure 2 illustrates the results of the screening of the DNA of the starting library (SL), selection cycle 12 (R12), aptamer sequences SP1-8 and SP5sc targeting SARS-CoV-2 spike. As can be taken from Figure 2, binding was obtained for the DNA of cycle 12 as well as for aptamers SP5, SP6, SP7 and SP8, whereas no binding was detectable for the DNA of the starting library as well as for the sequences of SP1-4 and the non-binding scrambled control sequences SP5sc. This shows the binding of the aptamers SP5, SP6, SP7 and SP8, corresponding to SEQ ID NOs: 18, 12, 19, 20, respectively, to SARS-CoV-2 spike glycoprotein.

### Example 4

### Determination of aptamer binding targeting spike proteins of SARS-CoV-2 and SARS-CoV-1, RBD and ACE2

The binding of the aptamer sequences SP5-8, corresponding to SEQ ID NOs: 18, 12, 19, 20, respectively, and SP5sc targeting the spike proteins of SARS-CoV-2 or of SARS-CoV-1, RBD and ACE2 immobilized on magnetic beads was investigated by FACS analysis according to example 2 do determine the specificity of the obtained aptamers.

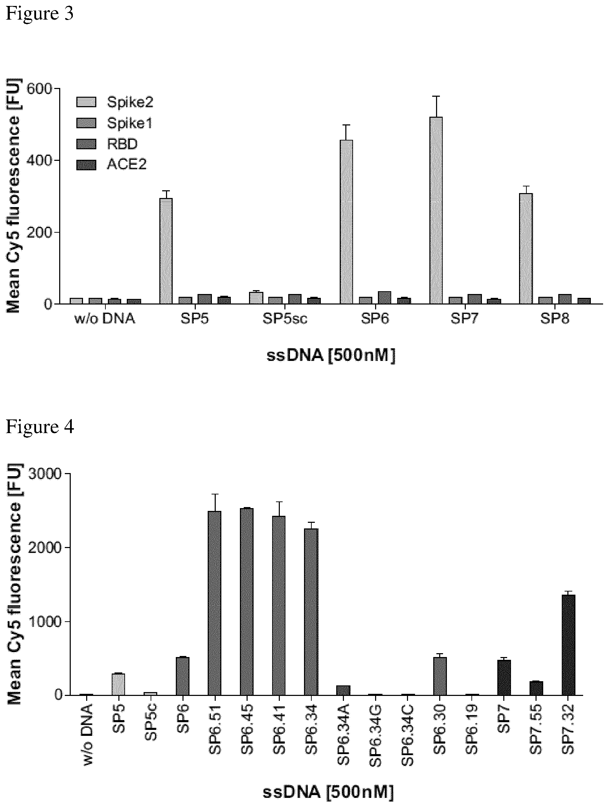

Figure 3 illustrates the results of the screening of the sequences SP5-8 and SP5sc targeting the spike proteins of SARS-CoV-2 (Spike2) or of SARS-CoV-1 (Spike1), RBD and ACE2. Binding was obtained specifically for sequences SP5, SP6, SP7 and SP8 targeting Spike2, whereas no binding was detectable for the control sequences SP5sc or any sequence targeting Spike1, RBD or ACE2.

This shows that the aptamers SP5, SP6, SP7 and SP8, corresponding to SEQ ID NOs: 18, 12, 19, 20, respectively, do not bind to the receptor-binding domain of the SARS-CoV-2 spike glycoprotein.

### Example 5

### Determination of aptamer binding targeting SARS-CoV-2 spike glycoprotein

The binding of the aptamers SP5-7, truncated aptamers SP6.51, SP6.45, SP6.41, SP6.34, SP6.30, SP6.19, SP7.55, SP7.32, sequences with one point mutation SP6.34A, SP6.34G and SP6.34C and SP5sc targeting SARS-CoV-2 spike glycoprotein immobilized on magnetic beads was investigated by FACS analysis according to example 2 to determine positions to truncate the aptamer and point mutations, which impair binding.

The following truncated aptamer sequences and point mutation sequences were used:
SP6.51: 5'-GATATCAACCCATGGTAGGTATTGCTTGGTAGGGATAGTGGGCTTGATGTT-3' (SEQ ID NO: 13)
SP6.45: 5'-ATCAACCCATGGTAGGTATTGCTTGGTAGGGATAGTGGGCTTGAT-3' (SEQ ID NO: 14)
SP6.41: 5'-CAACCCATGGTAGGTATTGCTTGGTAGGGATAGTGGGCTTG-3' (SEQ ID NO: 15)
SP6.34: 5'-CCCATGGTAGGTATTGCTTGGTAGGGATAGTGGG-3' (SEQ ID NO: 16)
SP6.30: 5'-CATGGTAGGTATTGCTTGGTAGGGATAGTG-3' (SEQ ID NO: 28)
SP6.19: 5'-TATTGCTTGGTAGGGATAG-3' (SEQ ID NO: 29)
SP7.32: 5'-GGAGGGTAGGTAGTGCTTGGTAGGGAAACTCC-3' (SEQ ID NO: 17)
SP7.55: 5'-GGAGGGAGATAGATATCAAAGGAGGGTAGGTAGTGCTTGGTAGGGAAACTCCGCC-3' (SEQ ID NO: 30)
SP6.34A: 5'-CCCATGGTAGGTATTGCATGGTAGGGATAGTGGG-3' (SEQ ID NO: 31),
SP6.34G: 5'-CCCATGGTAGGTATTGGTTGGTAGGGATAGTGGG-3' (SEQ ID NO: 32) and
SP6.34C: 5'-CCCATGGTAGGTATTGCTTGGTAGCGATAGTGGG-3' (SEQ ID NO: 33).

Figure 4 illustrates the results of the screening of the aptamers SP5-7, truncated aptamers SP6.51, SP6.45, SP6.41, SP6.34, SP6.30, SP6.19, SP7.55, SP7.32, sequences with one point mutation SP6.34A, SP6.34G and SP6.34C and SP5sc targeting SARS-CoV-2 spike glycoprotein. Binding was obtained for aptamers SP5, SP6 and SP7 as well as truncated aptamers SP6.51, SP6.45, SP6.41, SP6.34 and SP7.32 to a much higher extend as the full-length aptamers. No binding was detectable for the non-binding control sequence SP5sc, truncated aptamer SP6.19 and point mutants SP6.34G and SP6.34C, whereas impaired binding was detectable for truncated aptamer SP7.55 and point mutant SP6.34A. Without being bound to a specific theory, it is assumed that the three-dimensional stem-loop structure of the aptamer is impaired, particularly that a putative apical stem structure is destabilized.

This shows that the motif of SEQ ID NO: 1 is important for binding of the aptamers to SARS-CoV-2 spike glycoprotein. Further, a sequence length less then of the motif of SEQ ID NO: 1 lead to reduced binding or loss of binding capacity.

### Example 6

### SARS-CoV-2 spike glycoprotein detection by Sandwich ELONA

A Sandwich Enzyme-Linked OligoNucleotide Assay (ELONA) was performed to demonstrate SARS-CoV-2 spike glycoprotein detection using the full-length aptamer SP6 and truncated aptamer SP6.34 as well as non-binding control sequences SP6C (5'-GGGAGAGGAGGGAGATAGATATCAACCCATGGTAGGTATTGCTTGGTAGCGATAGTGGG CTTGATGTTTCGTGGATGCCACAGGAC-3' (SEQ ID NO: 34)) and SP6.34C.

33 ng/ml of nanobody VHH E (Nanobody Core Facility) was immobilized in 20 µl bicarbonate/carbonate buffer (pH 9.6) on 96 halve-well microtiter plates (MICROLON^{®} 600, VWR) at 4°C over night. After removal of the supernatant, two washing steps with 100 µl 1× PBS with 0.05% Tween 20 were performed. Wells were blocked with 1× PBS with 5% BSA for 1 hour at RT, followed by two washing steps with 100 µl 1× PBS with 3 mM MgCl₂. Afterwards the SARS-CoV-2 spike glycoprotein at concentrations of 1000, 500, 100, 50, 10, 5 and 1 nM in 1× PBS with 3 mM MgCl₂ and 0.8 mg/ml BSA was incubated in 20 µl at RT. After removal of the supernatant, two washing steps with 1× PBS with 3 mM MgCl₂ were performed. Next, 5'-biotinylated DNA aptamers SP6 and SP6.34 and controls SP6C and SP6.34C in 1× PBS with 3 mM MgCl₂ and 0.8 mg/ml BSA at a 500 nM concentration were incubated in 20 µl at RT, followed by two washing steps with 1× PBS with 3 mM MgCl₂. Streptavidin-HRP (GE Healthcare) in a 1:1000 dilution in 1× PBS with 3 mM MgCl₂ was incubated in 20 µl at RT, followed by two washing steps with 1× PBS with 3 mM MgCl₂. Finally, 100 µl ABTS (ThermoFisher) per well were incubated at RT for 40 min and the absorbance at 405 nm was measured on a Tecan Nanoquant (Tecan).

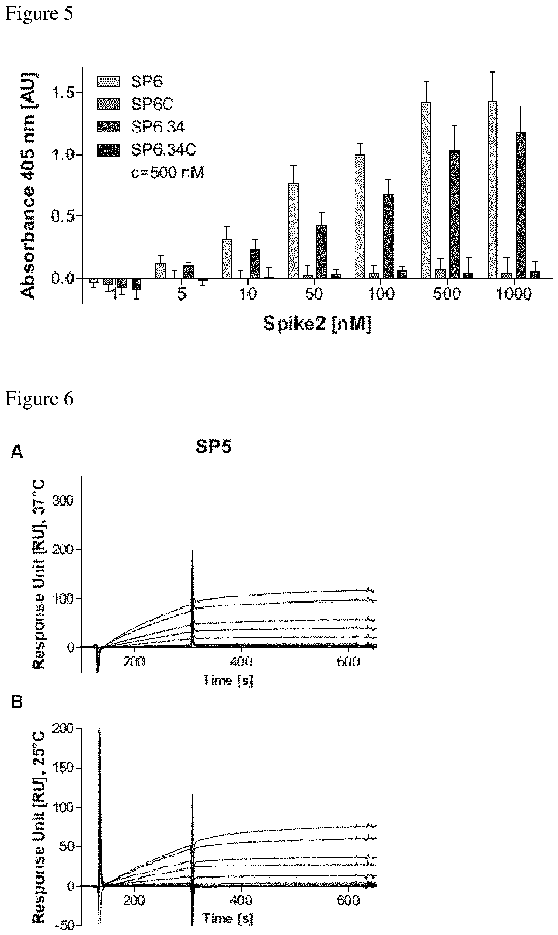

Figure 5 illustrates the results of the ELONA assay using the 5'-biotinylated aptamers SP6 and SP6.34, non-binding control sequences SP6C and SP6.34C and SARS-CoV-2 spike glycoprotein (Spike2) at concentrations of 1000, 500, 100, 50, 10, 5 and 1 nM. It was possible to detect Spike2 concentrations of 1000, 500, 100, 50, 10 and 5 nM using SP6 and SP6.34, whereas no detection was possible for a SARS-CoV-2 spike glycoprotein concentration of 1 nM or for the non-binding control sequences SP6C and SP6.34 at any SARS-CoV-2 spike glycoprotein concentration.

### Example 7

### K_{D} determination using surface plasmon resonance

Affinities of the 5'-biotinylated full-length aptamers SP5, SP6 and SP7 were determined by surface plasmon resonance (SPR) on a BIAcore 3000 instrument (GE Healthcare Europe). All buffers were filtered and degassed prior use. 50 nM biotinylated aptamers SP5 (flow cell 2), SP6 (flow cell 3) and SP7 (flow cell 4) and the control SP5sc (flow cell 1) in 0.5 M NaCl were immobilized on XanTec SAD chips (XanTec Bioanalytics) with a flow rate of 10 µl/min at 25 °C until a response of ∼200 response units was reached. The SARS-CoV-2 spike glycoprotein protein in 1× PBS with 3 mM MgCl₂ and 1 mg/ml BSA was injected at concentrations of 1000, 700, 316, 200, 100, 31.6, 10, 3.16 and 1 nM for 180 s at 25 °C and 37°C. The dissociation time was 400 s, followed by a regeneration step with 0.5% sodium dodecyl sulfate. After each regeneration step a new injection of SARS-CoV-2 spike glycoprotein at a higher concentrations followed up to 1000 nM. Data was evaluated using the BIAevaluation 4.1 (Biacore) software: 1:1 binding with drifting baseline.

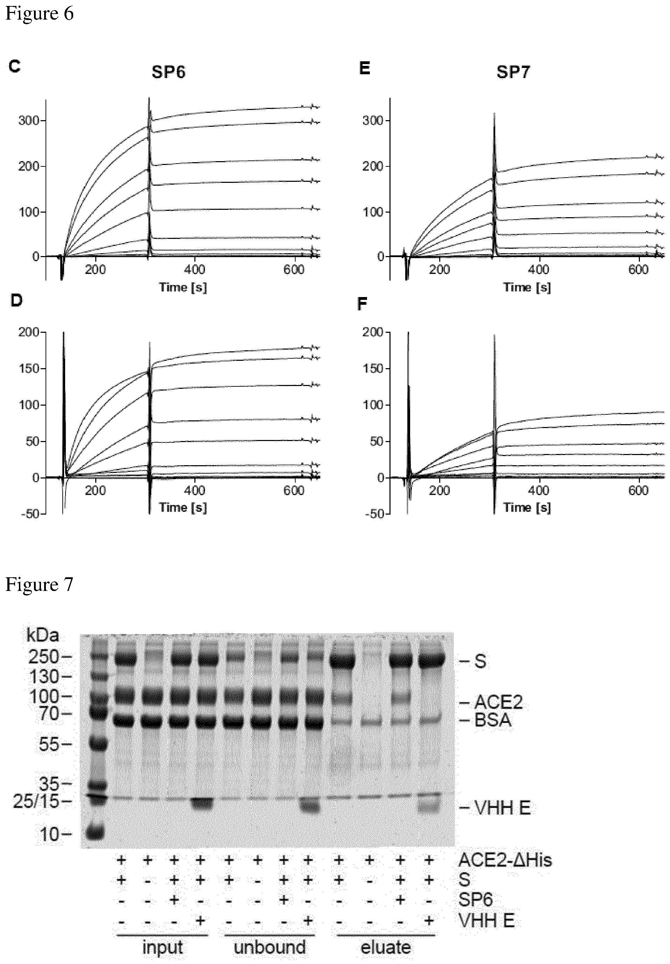

Figure 6 illustrates the results of the affinity determination by SPR for 5'-biotinylated full-length aptamers SP5, SP6 and SP7 at 37°C and 25°C in Figure 6 A), C) and E) and Figure 6 B), D) and F), respectively. After injection start of the SARS-CoV-2 spike glycoprotein (Spike2) at concentrations of 1000, 700, 316, 200, 100, 31.6, 10, 3.16 and 1 nM after 120 sec, an increase of response can be detected for SP5, SP6 and SP7 at both temperatures for the respective flow cell minus the response for the reference flow cell 1, which bears the non-binding control SP5sc. During SARS-CoV-2 spike glycoprotein injection, no plateau area was reached for any of the sequences at both temperatures. After injection stop after 300 sec and only 1× PBS with 3 mM MgCl₂ buffer flow, no reduction of response was detected, which would be caused by dissociation of the Spike2-aptamer complex.

The following Table 1 summarises the K_{D}, kₐ and k_{d} rates of SP5, SP6 and SP7 at 37°C and 25°C.

**Table 1: Binding and affinity constants of aptamers SP5 (SEQ ID NO: 18), SP6 (SEQ ID NO: 12) and SP7 (SEQ ID NO: 19) with different modifications**

| Sequence | temperature | kₐ (10⁴M⁻¹s⁻¹) | K_{d} (10⁴s⁻¹) | K_{D} [nM] |
|---|---|---|---|---|
| SP5 | 37 °C | 1 ± 0.2 | 37 ± 36 | 9.2 ± 7.9 |
| SP6 | 37 °C | 2.1 ± 0.6 | 4.5 ± 1.9 | 21 ± 4.6 |
| Sp7 | 37 °C | 1.5 ± 0.4 | 2.9 ± 1.2 | 18.9 ± 5.5 |
| SP5 | 25 °C | 1.19 ± 0.08 | 1.74 ± 0.01 | 14.7 ± 0.8 |
| SP6 | 25 °C | 2.5 ± 0.3 | 3.4 ± 0.2 | 13.9 ± 0.6 |
| Sp7 | 25 °C | 1.3 ± 0.2 | 1.7 ± 0.7 | 13.1 ± 3.8 |

This shows that the aptamers SP5, SP6 and SP7 bind with good affinity to SARS-CoV-2 spike glycoprotein. Further, a low k_{off} rate was seen.

### Example 8

### Determination that binding of ACE2 to SARS-CoV-2 spike glycoprotein not inhibited by aptamer SP6

As aptamer SP6 appears not to bind to the RBD of SARS-CoV-2 spike glycoprotein it was tested whether SP6 would interfere with binding of SARS-CoV-2 spike glycoprotein to ACE2. To this end His-tagged SARS-CoV-2 spike glycoprotein was pulled by Ni-NTA magnetic beads and co-pulldown of ACE2 lacking the His tag (ACE2ΔHis) was analyzed.

For spike-ACE2 complex pulldowns 1 µM SARS-CoV-2-S-HexaPro and ACE2 (without 8xHis tag) were incubated in the presence of 3 µM SP6 or VHH E, as indicated, in PBS / 4 mM MgCl₂ / 2.5 µM BSA. An aliquot was removed (input) and 20 µl pre-equilibrated HisPur Ni-NTA Magnetic Beads (Thermo) were added. After 30 min incubation at room temperature (rt) on an overhead rotator beads were collected on a magnet and an aliquot was removed from the supernatant (unbound). After washing two times with 250 µl PBS / 3 mM MgCl₂ proteins were eluted with 25 mM HEPES/KOH, pH 7.8 / 150 mM NaCl / 1 M imidazole (eluate). Samples were separated by SDS-PAGE. Coomassie-stained gels were scanned on an Odyssey Sa (Licor).

Figure 7 shows the Coomassie-stained gels illustrating that whereas the interaction between SARS-CoV-2 spike glycoprotein and ACE2 was abolished by nanobody VHH E it was not affected by aptamer SP6. Densitometry of the bands gave ratios of ACE2 : S of 0.18 and 0.16 in the absence or presence of SP6, respectively. This demonstrates that aptamer SP6 does not affect binding of SARS-CoV-2 spike protein to ACE2.

### Example 9

### Determination of inhibition of pseudovirus infection

To address the question whether SP6 would interfere with infection the established VSV-AG-based pseudotype system was used which allows infection being quantified by flow cytometry. ACE2-expressing Vero E6 cells were infected with Cov2-S or VSV-G pseudotyped viruses which had been pre-incubated with aptamers or not as detailed below. Pseudotype particle number was adjusted such that the infection rate of untreated pseudotypes was between 8 % and 10 %. The low infection rate was chosen to prevent multiple infections of a single cell which would preclude reliable measurements.

Vero E6 cells were cultivated in DMEM (Thermo) / 10 % FBS (PAN) at 37 °C and 8 % CO₂. The day before infection 5x10⁴ cells were plated per well of a 24well plate. Virus particles pseudotyped with SARS-CoV-2-S(D614G)-Δ19 or VSV-G were pre-incubated for 20 min at room temperature (rt) with the indicated aptamer in DMEM / 3 mM MgCl₂. The culture medium was removed from the cells and replaced by 150 µl pre-incubated virus (MOI ≈ 0.1). After incubation for 1 h at 37 °C 0.5 ml DMEM / 10 % FBS / 3 mM MgCl₂ was added and the cells were cultivated for 16 - 18 h. Cells were detached with trypsin, fixed for 20 min at rt with 4 % formaldehyde, pelleted (800 g, 5 min, rt) and resuspended in PBS. Infection rate was determined as percentage of GFP-positive cells by flow cytometry (BectonDickinson). For statistical analysis the non-parametric Kruskal-Wallis test and Dunn's multiple comparison post-test was used because due to the small sample size of n=5 Gaussian distribution fo the values could not be tested. Analysis was performed with Prism 5.0f (GraphPad).

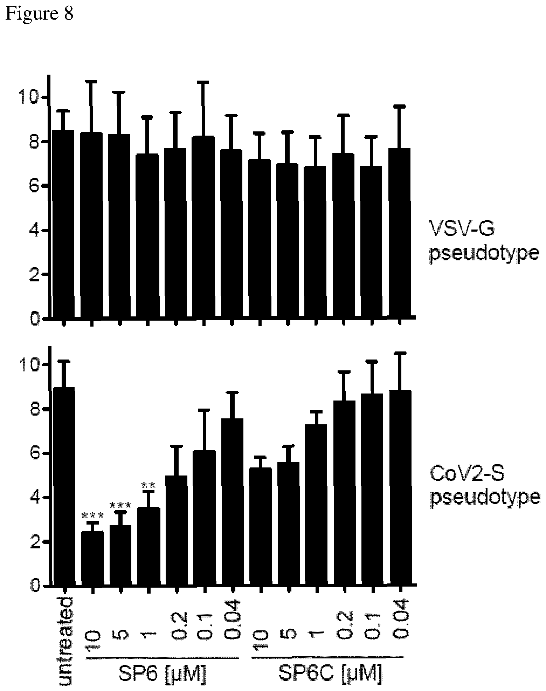

Figure 8 illustrates the infection results. As can be taken from the Figure 8, SP6 showed a concentration-dependent reduction of infection by CoV2-S pseudotype. In contrast, VSV-G pseudotype was not affected demonstrating that the inhibitory effect of SP6 depends on the presence of CoV2-S on the viral particles and excluding an unspecific effect on the infection process of the VSV backbone virus. SP6C showed some reduction of infection in agreement with its reduced binding to CoV2-S in the pulldown assay. Inhibition by SP6C did not reach statistical significance but also depended on the presence of CoV2-S on the viral particles. n=5, *** p<0.001, ** p<0.01, numbers on the y-axis represent percent infected cells.

This shows that the aptamer provides a reduction of infection by CoV2-S pseudotype and thus could be usable in the treatment of a SARS-CoV-2 infection.

The work leading to this invention has received funding from BMBF under grant agreement n° 01KI20154.

## Claims

1. An aptamer binding to SARS-CoV-2 spike glycoprotein, wherein the aptamer does not bind to the receptor-binding domain of the SARS-CoV-2 spike glycoprotein nor inhibit the interaction of the receptor-binding domain (RBD) of SARS-CoV-2 spike glycoprotein with angiotensin-converting enzyme II (ACE2) and/or comprises or consists of a nucleotide sequence 5'-N₁N₂AN₃GGTAGGTAN4TGCTTGGTAGGGAN₅AN₆TN₇N₈GN₉N₁₀-3' (SEQ ID NO: 1) or a pharmaceutically acceptable salt thereof, wherein:
N₁, N₂ independently represent G or C,
N₃, N₄ independently represent T or G,
N₅ represents T or A,
N₆, N₇, N₈ independently represent G or C, and
N₉, N₁₀ independently represent C or T.

2. The aptamer according to claim 1, wherein the aptamer comprises or consists of a nucleotide sequence 5'-CCATGGTAGGTATTGCTTGGTAGGGATAGTGGGCT-3' (SEQ ID NO: 2) or 5'-GGAGGGTAGGTAGTGCTTGGTAGGGAAACTCCGCC-3' (SEQ ID NO: 3) or a pharmaceutically acceptable salt thereof.

3. The aptamer according to according to claim 1 or 2, wherein the aptamer comprises or consists of a nucleotide sequence or a pharmaceutically acceptable salt thereof selected from the group comprising:
5'-CCCATGGTAGGTATTGCTTGGTAGGGATAGTGGGCTTGATG-3' (SEQ ID NO: 4),
5'-CCATGGTAGGTATTGCTTGGTAGGGATAGTGGGCTTGATG-3' (SEQ ID NO: 5), and
5'-CCCATGGTAGGTATTGCTTGGTAGGGATAGTGGGTTTGATG-3' (SEQ ID NO: 6).

4. The aptamer according to any of claims 1 or 2, wherein the aptamer comprises or consists of a nucleotide sequence or a pharmaceutically acceptable salt thereof selected from the group comprising:
5'-AGGAGGGTAGGTAGTGCTTGGTAGGGAAACTCCGCCGATT-3' (SEQ ID NO: 7),
5'-AAGGAGGGTAGGTAGTGCTTGGTAGGGAAACTCCGCCGATT-3' (SEQ ID NO: 8),
5'-AAGGAGGGTAGGTAGTGCTTGGTAGGGAAACTCCGCCGAT-3' (SEQ ID NO: 9),
5'-AGGAGGGTAGGTAGTGCTTGGTAGGGAAACTCCGTCGATT-3' (SEQ ID NO: 10), and
5'-AAGGAGGGTAGGTAGTGCTTGGTAGGGAAACTCCGTCGAT-3' (SEQ ID NO: 11).

5. The aptamer according to any of claims 1 to 3, wherein the aptamer comprises or consists of a nucleotide sequence selected from the group comprising: 5'-GGGAGAGGAGGGAGATAGATATCAACCCATGGTAGGTATTGCTTGGTAGGGATA GTGGGCTTGATGTTTCGTGGATGCCACAGGAC-3' (SEQ ID NO: 12), 5'-GATATCAACCCATGGTAGGTATTGCTTGGTAGGGATAGTGGGCTTGATGTT-3' (SEQ ID NO: 13), 5'-ATCAACCCATGGTAGGTATTGCTTGGTAGGGATAGTGGGCTTGAT-3' (SEQ ID NO: 14),
5'-CAACCCATGGTAGGTATTGCTTGGTAGGGATAGTGGGCTTG-3' (SEQ ID NO: 15) and
5'-CCCATGGTAGGTATTGCTTGGTAGGGATAGTGGG-3' (SEQ ID NO: 16) or a pharmaceutically acceptable salt thereof.

6. The aptamer according to any of claims 1 to 5, wherein the aptamer is part of a dimer, trimer or multimer comprising two, three or multiple nucleotide sequences selected from the group comprising SEQ ID NOs: 1-20, wherein the nucleotide sequences are connected by a linker, which linker preferably is selected from a nucleotide linker, polyethylene glycole, a peptide linker, an alkyl linker or an aminoalkyl linker.

7. An aptamer according to anyone of claims 1 to 6, for use as a medicament or a diagnostic reagent.

8. The aptamer for use according to claim 7, wherein the aptamer is for use in the detection of SARS-CoV-2 or diagnosis or treatment of a SARS-CoV-2 infection.

9. A diagnostic or pharmaceutical composition comprising an aptamer according to anyone of claims 1 to 6.

10. The diagnostic or pharmaceutical composition according to claim 9 for use in the detection of SARS-CoV-2 or diagnosis or treatment of a SARS-CoV-2 infection.

11. A diagnostic composition according to claim 9 or 10, wherein the diagnostic composition is used in an enzyme-linked oligonucleotide assay (ELONA) or in a lateral flow assay (LFA).

12. The pharmaceutical composition according to claim 9 or 10, wherein in the pharmaceutical composition is in form of a nasal spray.

13. Use of an aptamer according to anyone of the claims 1 to 6, for the manufacture of a medicament or a diagnostic reagent, particularly for use in the detection of SARS-CoV-2 or diagnosis or treatment of a SARS-CoV-2 infection.

14. An *in vitro* method of detecting SARS-CoV-2 or diagnosing a SARS-CoV-2 infection, the method comprising the step of detecting the binding of an aptamer according to anyone of the claims 1 to 6 to SARS-CoV-2 Spike glycoprotein in a sample obtained from a subject.

15. A method of treating a SARS-CoV-2 infection, the method comprising the step of administering to a subject a therapeutically effective amount of an aptamer according to anyone of the claims 1 to 6.


SEQUENCE LISTING

<110>  UD 41559/SAM

<120>  Aptamers and use of the aptamers in the diagnosis and treatment
       of a SARS-CoV-2 infection

<130>  UD 41559/SAM

<160>  34

<170>  PatentIn version 3.3

<210>  1
<211>  35
<212>  DNA
<213>  artificial

<220>
<223>  aptamer



<220>
<221>  misc_feature
<222>  (1)..(1)
<223>  n is g or c

<220>
<221>  misc_feature
<222>  (2)..(2)
<223>  n is g or c

<220>
<221>  misc_feature
<222>  (4)..(4)
<223>  n is t or g

<220>
<221>  misc_feature
<222>  (13)..(13)
<223>  n is t or g

<220>
<221>  misc_feature
<222>  (27)..(27)
<223>  n is t or a

<220>
<221>  misc_feature
<222>  (29)..(29)
<223>  n is g or c

<220>
<221>  misc_feature
<222>  (31)..(31)
<223>  n is g or c

<220>
<221>  misc_feature
<222>  (32)..(32)
<223>  n is g or c

<220>

<221>  misc_feature
<222>  (34)..(34)
<223>  n is c or t

<220>
<221>  misc_feature
<222>  (35)..(35)
<223>  n is c or t

<400>  1
nnanggtagg tantgcttgg taggganant nngnn                                35


<210>  2
<211>  35
<212>  DNA
<213>  Artificial

<220>
<223>  aptamer

<400>  2
ccatggtagg tattgcttgg tagggatagt gggct                                35


<210>  3
<211>  35
<212>  DNA
<213>  Artificial

<220>
<223>  aptamer

<400>  3
ggagggtagg tagtgcttgg tagggaaact ccgcc                                35


<210>  4
<211>  41
<212>  DNA
<213>  Artificial

<220>
<223>  aptamer

<400>  4
cccatggtag gtattgcttg gtagggatag tgggcttgat g                         41


<210>  5
<211>  40
<212>  DNA
<213>  Artificial

<220>
<223>  aptamer

<400>  5
ccatggtagg tattgcttgg tagggatagt gggcttgatg                           40


<210>  6
<211>  41

<212>  DNA
<213>  Artificial

<220>
<223>  aptamer

<400>  6
cccatggtag gtattgcttg gtagggatag tgggtttgat g                          41


<210>  7
<211>  40
<212>  DNA
<213>  Artificial

<220>
<223>  aptamer

<400>  7
aggagggtag gtagtgcttg gtagggaaac tccgccgatt                            40


<210>  8
<211>  41
<212>  DNA
<213>  Artificial

<220>
<223>  aptamer

<400>  8
aaggagggta ggtagtgctt ggtagggaaa ctccgccgat t                          41


<210>  9
<211>  40
<212>  DNA
<213>  Artificial

<220>
<223>  aptamer

<400>  9
aaggagggta ggtagtgctt ggtagggaaa ctccgccgat                            40


<210>  10
<211>  40
<212>  DNA
<213>  Artificial

<220>
<223>  aptamer

<400>  10
aggagggtag gtagtgcttg gtagggaaac tccgtcgatt                            40


<210>  11
<211>  40
<212>  DNA
<213>  Artificial

<220>
<223>  aptamer

<400>  11
aaggagggta ggtagtgctt ggtagggaaa ctccgtcgat                          40


<210>  12
<211>  86
<212>  DNA
<213>  Artificial

<220>
<223>  aptamer

<400>  12
gggagaggag ggagatagat atcaacccat ggtaggtatt gcttggtagg gatagtgggc    60

ttgatgtttc gtggatgcca caggac                                         86


<210>  13
<211>  51
<212>  DNA
<213>  Artificial

<220>
<223>  aptamer

<400>  13
gatatcaacc catggtaggt attgcttggt agggatagtg ggcttgatgt t             51


<210>  14
<211>  45
<212>  DNA
<213>  Artificial

<220>
<223>  aptamer

<400>  14
atcaacccat ggtaggtatt gcttggtagg gatagtgggc ttgat                    45


<210>  15
<211>  41
<212>  DNA
<213>  Artificial

<220>
<223>  aptamer

<400>  15
caacccatgg taggtattgc ttggtaggga tagtgggctt g                        41


<210>  16
<211>  34
<212>  DNA
<213>  Artificial

<220>

<223>  aptamer

<400>  16
cccatggtag gtattgcttg gtagggatag tggg                                34


<210>  17
<211>  32
<212>  DNA
<213>  Artificial

<220>
<223>  aptamer

<400>  17
ggagggtagg tagtgcttgg tagggaaact cc                                  32


<210>  18
<211>  85
<212>  DNA
<213>  Artificial

<220>
<223>  aptamer

<400>  18
gggagaggag ggagatagat atcaaccatg gtaggtattg cttggtaggg atagtgggct    60

tgatgtttcg tggatgccac aggac                                          85


<210>  19
<211>  85
<212>  DNA
<213>  Artificial

<220>
<223>  aptamer

<400>  19
gggagaggag ggagatagat atcaaaggag ggtaggtagt gcttggtagg gaaactccgc    60

cgatttttcg tggatgccac aggac                                          85


<210>  20
<211>  86
<212>  DNA
<213>  Artificial

<220>
<223>  aptamer

<400>  20
gggagaggag ggagatagat atcaaaagga gggtaggtag tgcttggtag ggaaactccg    60

ccgatttttc gtggatgcca caggac                                         86


<210>  21
<211>  85
<212>  DNA

<213>  Artificial

<220>
<223>  library


<220>
<221>  misc_feature
<222>  (26)..(65)
<223>  n is a, c, t or g

<400>  21
gggagaggag ggagatagat atcaannnnn nnnnnnnnnn nnnnnnnnnn nnnnnnnnnn      60

nnnnntttcg tggatgccac aggac                                            85


<210>  22
<211>  25
<212>  DNA
<213>  Artificial

<220>
<223>  primer

<400>  22
gggagaggag ggagatagat atcaa                                            25


<210>  23
<211>  20
<212>  DNA
<213>  Artificial

<220>
<223>  primer

<400>  23
gtcctgtggc atccacgaaa                                                  20


<210>  24
<211>  85
<212>  DNA
<213>  Artificial

<220>
<223>  aptamer

<400>  24
gggagaggag ggagatagat atcaagggcg ggagggaggg gggccacacc aaaacacgtt      60

caacttttcg tggatgccac aggac                                            85


<210>  25
<211>  85
<212>  DNA
<213>  Artificial

<220>
<223>  aptamer

<400>  25
gggagaggag ggagatagat atcaagggag ggagggtggg gggttctcgc tgcgggtttt      60

ggtgctttcg tggatgccac aggac                                            85


<210>  26
<211>  85
<212>  DNA
<213>  Artificial

<220>
<223>  aptamer

<400>  26
gggagaggag ggagatagat atcaaatcgg ggggtgggtt tgggtatggg gtctgcacta      60

tggcttttcg tggatgccac aggac                                            85


<210>  27
<211>  85
<212>  DNA
<213>  Artificial

<220>
<223>  aptamer

<400>  27
gggagaggag ggagatagat atcaatcgcg gggggcgggt cgggtgctcg ttcgaggggt      60

cgcagtttcg tggatgccac aggac                                            85


<210>  28
<211>  30
<212>  DNA
<213>  Artificial

<220>
<223>  aptamer

<400>  28
catggtaggt attgcttggt agggatagtg                                       30


<210>  29
<211>  19
<212>  DNA
<213>  Artificial

<220>
<223>  aptamer

<400>  29
tattgcttgg tagggatag                                                   19


<210>  30
<211>  55
<212>  DNA
<213>  Artificial

<220>
<223>  aptamer

<400>  30
ggagggagat agatatcaaa ggagggtagg tagtgcttgg tagggaaact ccgcc          55


<210>  31
<211>  34
<212>  DNA
<213>  Artificial

<220>
<223>  aptamer

<400>  31
cccatggtag gtattgcatg gtagggatag tggg                                 34


<210>  32
<211>  34
<212>  DNA
<213>  Artificial

<220>
<223>  aptamer

<400>  32
cccatggtag gtattggttg gtagggatag tggg                                 34


<210>  33
<211>  34
<212>  DNA
<213>  Artificial

<220>
<223>  aptamer

<400>  33
cccatggtag gtattgcttg gtagcgatag tggg                                 34


<210>  34
<211>  86
<212>  DNA
<213>  Artificial

<220>
<223>  aptamer

<400>  34
gggagaggag ggagatagat atcaacccat ggtaggtatt gcttggtagc gatagtgggc     60

ttgatgtttc gtggatgcca caggac                                          86